# FASCICULE DE BREVET EUROPEEN

(11) **EP 4 094 731 B1**
(45) Date de publication et mention de la délivrance du brevet: **04.03.2026**
(21) Numéro de dépôt: 22175848.5
(22) Date de dépôt: 27.05.2022
(51) Int. Cl.: A61F 5/01, B25J 9/00, A61H 3/00, A61H 1/02

(54) **DISPOSITIF ORTHOPEDIQUE COMPRENANT AU MOINS UN ACTIONNEUR**
ORTHOPÄDISCHE VORRICHTUNG, DIE MINDESTENS EIN STELLORGAN UMFASST
ORTHOPAEDIC DEVICE COMPRISING AT LEAST ONE ACTUATOR

(30) Priorité: 27.05.2021 FR 2105545
(43) Date de publication de la demande: 30.11.2022
(73) Titulaire: Reev SAS, 31670 Labège (FR)
(72) Inventeur: TEMPORELLI, Robin, 31000 TOULOUSE (FR)
(74) Mandataire: Cabinet Chaillot

(56) Documents cités:
- US-A1- 2014 358 053
- US-A1- 2015 226 234
- US-A1- 2019 070 061
- US-A1- 2020 069 441
- US-B2- 10 517 788

## Description

### Domaine technique

L'invention concerne un dispositif orthopédique comprenant au moins un actionneur. L'invention concerne en particulier un tel dispositif orthopédique pour un membre inférieur humain.

Un dispositif orthopédique selon l'invention est utile pour des personnes présentant des troubles moteurs légers (momentanés ou définitifs), par exemple des personnes atteintes de certaines pathologies neurologiques, des personnes atteintes par certaines pathologies ostéoarticulaires ou encore des personnes présentant une atrophie musculaire momentanée. L'invention peut aussi être utile pour des personnes nécessitant une assistance musculaire pour effectuer un travail et notamment ce qui est parfois appelé le renfort terrain.

Dans tout le texte, on entend par "dispositif orthopédique", tout dispositif destiné à soutenir un membre humain, et/ou à pallier les déficiences fonctionnelles d'un membre humain, ou, éventuellement, tout dispositif destiné à remplacer au moins partiellement un membre humain.

### État de la technique

On connaît des orthèses jambières dites actives permettant de motoriser en partie la flexion et/ou l'extension d'une jambe.

WO 2016/171548 décrit par exemple un actionneur prothétique comprenant un vérin hydraulique ou pneumatique connecté à une partie supérieure et à une partie inférieure d'une jambe, une vanne automatique, un accumulateur d'énergie et un capteur de détection d'un déplacement.

Les dispositifs orthopédiques connus sont généralement lourds et encombrants, la masse et le volume de tels dispositifs étant souvent mal répartis sur le corps de l'utilisateur.

De tel dispositifs orthopédiques ne présentent pas d'orthèses réalisées sur-mesure et ne s'adaptent pas au handicap et à la morphologie de l'utilisateur.

De tel dispositifs orthopédiques sont également dispendieux et ne restent accessible qu'à une élite ou à certains centres de rééducation spécialisés. Ainsi, l'assistance et/ou la rééducation et/ou l'aide sur-mesure et journalière par dispositif orthopédique actif reste aujourd'hui un besoin peu et mal adressé chez l'Homme alors que la demande est croissante.

L'invention vise à pallier l'ensemble de ces inconvénients.

L'invention vise à proposer un dispositif orthopédique selon l'invention peu invasif et ergonomique (léger et peu encombrant) pour un utilisateur.

L'invention vise également à proposer un certain équilibre dans la répartition de la masse et du volume du dispositif orthopédique sur le corps afin de le rendre confortable pour l'utilisateur.

L'invention vise également à proposer un tel dispositif orthopédique particulièrement discret lorsqu'il est porté par un utilisateur.

L'invention vise également à proposer un tel dispositif orthopédique sur-mesure et donc adapté au handicap et à la morphologie de l'utilisateur.

L'invention vise également à proposer un tel dispositif orthopédique présentant un coût limité afin de le rendre accessible au plus grand nombre et pour une utilisation quotidienne.

L'invention vise également à proposer un tel dispositif orthopédique utile pour des utilisateurs d'âges variés et présentant une grande diversité de pathologies ou de besoin d'assistance et résistance moteur.

Un aspect non revendiqué de l'invention concerne un procédé de mise en œuvre d'un tel dispositif orthopédique.

Les documents US 2020/069441 A1, US 10 517 788 B2, US 2019/070061 A1, US 2015/226234 A1 et US 2014/358053 A1 illustrent l'état de la technique pour un dispositif orthopédique selon la revendication 1.

### Exposé de l'invention

L'invention est définie dans la revendication 1 et concerne un dispositif orthopédique, en particulier pour un membre inférieur humain, comprenant :
- au moins une portion, dite exosquelette supérieur, adaptée pour former un appui en contact avec une première partie du corps humain,
- au moins une portion, dite exosquelette inférieur, adaptée pour former un appui en contact avec une deuxième partie du corps humain,
- un actionneur, dit actionneur récepteur, comprenant :
   o un organe de liaison pivot présentant au moins un axe de pivot, ledit exosquelette supérieur et ledit exosquelette inférieur étant articulés l'un par rapport à l'autre par ledit organe de liaison pivot,
   o un dispositif de transmission récepteur adapté pour pouvoir transmettre un mouvement à l'organe de liaison pivot, et
   o au moins un premier vérin hydraulique, dit premier vérin récepteur, accouplé au dispositif de transmission récepteur pour pouvoir entraîner en rotation l'organe de liaison pivot,
- un actionneur, dit actionneur émetteur, comprenant :
   o un dispositif moteur, et
   o deux vérins hydrauliques, dit respectivement premier vérin émetteur et deuxième vérin émetteur, comprenant chacun un piston et un cylindre associé, et
- deux conduits hydrauliques de guidage de fluide sous pression respectivement reliés au premier vérin émetteur et au deuxième vérin émetteur, et adaptés pour permettre une transmission hydraulique de mouvement de l'actionneur émetteur à l'actionneur récepteur,
le dispositif moteur est accouplé au dispositif de transmission émetteur de type vis/écrou, un rotor du dispositif moteur étant couplé à la vis du dispositif de transmission émetteur, cette vis coopérant avec le taraudage d'un écrou relié mécaniquement aux pistons respectifs du premier vérin émetteur hydraulique et du deuxième vérin émetteur hydraulique ; et le premier vérin émetteur et le deuxième vérin émetteur sont installés en opposition en étant reliés mécaniquement à l'écrou du dispositif de transmission émetteur de telle sorte que lorsque ledit écrou se déplace en translation par l'action de rotation de la vis du dispositif de transmission émetteur le piston du premier vérin émetteur rentre dans le cylindre associé et le piston du deuxième vérin émetteur hydraulique sort du cylindre associé, et vice versa.

Un dispositif orthopédique selon l'invention permet ainsi, notamment grâce à l'utilisation d'un tel conduit hydraulique de guidage de fluide sous pression, de pouvoir déporter l'actionneur émetteur par rapport à l'actionneur récepteur, c'est-à-dire l'unité de puissance du dispositif orthopédique par rapport à une articulation humaine telle que le genou, la cheville ou encore la hanche. Cela permet de diminuer la masse du dispositif orthopédique supportée par l'utilisateur au niveau du membre inférieur lui-même ainsi que son encombrement (son volume). En effet, il est ainsi possible de positionner l'ensemble formé par l'actionneur émetteur, non pas au niveau du membre inférieur mais par exemple à la ceinture ou encore dans un sac à dos porté par l'utilisateur. Les inventeurs ont observé que cela permet d'améliorer les sensations d'équilibre et de confort de l'utilisateur de sorte que ses mouvements sont facilités et plus naturels.

Le mécanisme électrohydraulique comprenant l'ensemble formé par ledit actionneur récepteur, ledit actionneur émetteur et ledit au moins un conduit de guidage de fluide sous pression est adapté pour pouvoir se connecter mécaniquement avec différentes conceptions desdits exosquelette supérieur et exosquelette inférieur.

Dans le cas où le dispositif orthopédique est une orthèse pour le genou, l'exosquelette supérieur peut être disposé au moins en partie autour de la cuisse dudit membre et l'exosquelette inférieur peut être disposé au moins en partie autour du mollet dudit membre.

Selon certains modes de réalisation, ledit dispositif orthopédique selon l'invention est une orthèse, en particulier une orthèse destinée à être utilisée pour équiper la jambe d'un utilisateur de façon à assister l'articulation formée par le genou. Le dispositif orthopédique selon l'invention peut ainsi assister les mouvements de flexion et d'extension d'une jambe.

Selon certains modes de réalisation, ledit exosquelette inférieur comprend en outre une portion, dite portion plantaire, adaptée pour recevoir au moins en partie la plante du pied. Ainsi, ledit exosquelette inférieur peut être conçu de manière à rendre ledit dispositif orthopédique complètement ou partiellement relié à la terre, la masse dudit dispositif orthopédique est alors complètement ou partiellement autoportée.

Selon certains modes de réalisation, ledit dispositif orthopédique selon l'invention peut être adapté pour pouvoir équiper une orthèse existante, l'orthèse existante formant alors ledit exosquelette supérieur et/ou ledit exosquelette inférieur. Le dispositif orthopédique selon l'invention peut donc être accommodé à une telle orthèse, ou encore à une prothèse.

Selon certains modes de réalisation, ledit premier vérin émetteur dudit actionneur émetteur est un vérin à simple effet. Selon certains modes de réalisation, ledit premier vérin émetteur dudit actionneur émetteur est un vérin à double effet.

Selon certains modes de réalisation, ledit deuxième vérin émetteur dudit actionneur émetteur est un vérin à simple effet. Selon certains modes de réalisation, ledit deuxième vérin émetteur dudit actionneur émetteur est un vérin à double effet.

Chaque actionneur émetteur comprend au moins un dispositif moteur générateur d'énergie mécanique, qui peut être de différentes natures : par exemple un moteur électrique et/ou un moteur hydraulique et/ou un moteur pneumatique, etc.

Selon certains modes de réalisation, ledit dispositif moteur dudit actionneur émetteur est un moteur électrique. Il peut s'agir par exemple d'un moteur électrique alimenté par un accumulateur d'énergie électrique, tel qu'une batterie électrique, notamment une batterie électrique rechargeable.

Selon certains modes de réalisation, ledit actionneur récepteur comprend un deuxième vérin hydraulique, dit deuxième vérin récepteur, le premier vérin récepteur et ledit deuxième vérin récepteur étant respectivement reliés à chacun des deux conduits hydrauliques de guidage de fluide sous pression.

Selon certains modes de réalisation, ledit premier vérin récepteur dudit actionneur récepteur est un vérin à simple effet. Selon certains modes de réalisation, ledit premier vérin récepteur dudit actionneur récepteur est un vérin à double effet.

Selon certains modes de réalisation, ledit deuxième vérin récepteur dudit actionneur récepteur est un vérin à simple effet. Selon certains modes de réalisation, ledit deuxième vérin récepteur dudit actionneur récepteur est un vérin à double effet.

Selon certains modes de réalisation, ledit dispositif de transmission récepteur est choisi dans le groupe formé des transmissions mécaniques adaptées pour pouvoir transformer un mouvement de translation transmis par l'actionneur récepteur en un mouvement de rotation de l'organe de liaison pivot.

Selon certains modes de réalisation, ledit dispositif de transmission récepteur est choisi dans le groupe formé des transmissions mécaniques à câbles et/ou courroies, des transmissions de type bielle/manivelle et des transmissions mécaniques de type pignon/crémaillère.

Ledit organe de liaison pivot présente au moins un axe de rotation, dit axe de pivot. Ledit organe de liaison pivot peut présenter un ou plusieurs axes de rotation (axes monocentriques ou polycentriques). Selon certains modes de réalisation, ledit organe de liaison pivot est polycentrique. Un tel organe de liaison pivot polycentrique permet de mieux reproduire la cinématique du genou par exemple et contribue ainsi à améliorer l'ergonomie du dispositif orthopédique selon l'invention.

Selon certains modes de réalisation, ledit organe de liaison pivot comprend une roue dentée fixée à un levier relié audit exosquelette inférieur. Ledit levier peut se présenter sous la forme d'une barre présentant plusieurs portions courbes et/ou rectilignes juxtaposées entre elles.

Selon certains modes de réalisation, le levier dudit organe de liaison pivot est adapté pour autoriser une rotation d'au moins deux portions de ladite barre métallique l'une par rapport à l'autre autour d'un axe orthogonal audit axe de pivot. Ainsi, ledit organe de liaison peut également présenter un ou plusieurs degré(s) de réglage hors plan afin de mieux s'adapter à la démarche de l'utilisateur.

Comme on l'aura compris le dispositif orthopédique selon l'invention comprend deux conduits hydrauliques de guidage de fluide sous pression, en particulier chaque conduit étant relié à un vérin de chaque actionneur. Chaque conduit hydraulique de guidage de fluide sous pression permet une transmission de mouvement d'un vérin émetteur à un vérin récepteur.

Selon certains modes de réalisation, un dispositif orthopédique selon l'invention comprend au moins un capteur choisi dans le groupe formé des accéléromètres, des capteurs de position, des capteurs de pression, des capteurs de couple de torsion, des capteurs de force et des capteurs de déformation.

Selon certains modes de réalisation, un dispositif orthopédique selon l'invention comprend en outre une unité électronique pour définir une commande à appliquer à l'organe de liaison pivot et une interface homme/machine pour permettre à un utilisateur de définir des paramètres de fonctionnement du dispositif orthopédique. L'interface homme/machine peut comprendre tout type d'écran d'affichage. L'interface homme/machine permet à un utilisateur de définir les paramètres de fonctionnement du dispositif orthopédique mais aussi d'afficher certains bio-marqueurs caractéristiques de la bio-cinématique de l'homme ; exemple pour les membres inférieurs : type et nombre de cycles effectués (marche, montées d'escaliers, assis/debout), puissance mécanique développée ; exemple pour la marche en particulier: longueur de foulée, temps de contact, temps de double contact, temps de vol, nombre de pas, distance parcourue, vitesse, etc. L'unité électronique comprend un accumulateur d'énergie électrique, tel qu'une batterie électrique, par lequel elle est alimentée.

Chaque capteur est adapté pour transmettre des données représentatives du fonctionnement du dispositif orthopédique à ladite unité électronique. Le fonctionnement du dispositif orthopédique est commandé selon des boucles de contrôles prenant en compte ces données ainsi que les paramètres choisis par l'utilisateur via l'interface homme/machine.

L'interface homme/machine peut également comprendre une unité de connexion à au moins une liaison de communication de données numériques avec au moins un serveur distant.

Selon certains modes de réalisation, l'organe de liaison pivot peut comprendre une roue dentée et un levier qui est relié à l'exosquelette inférieur et qui se présente sous la forme d'une barre ayant plusieurs portions courbes et/ou rectilignes juxtaposées entre elles, ledit organe de liaison pivot étant adapté pour autoriser une rotation d'au moins deux portions de ladite barre métallique l'une par rapport à l'autre autour d'un axe orthogonal à l'axe de pivot de l'organe de liaison pivot de sorte que ledit organe de liaison pivot peut présenter un ou plusieurs degré(s) de réglage hors plan pour s'adapter à la démarche d'un utilisateur.

Selon certains modes de réalisation, l'organe de liaison pivot peut être un organe de liaison pivot polycentrique.

Selon certains modes de réalisation, l'organe de liaison pivot polycentrique peut être adapté à la cinématique d'une articulation entre la première partie et la deuxième partie du corps humain au contact desquelles l'exosquelette supérieur et l'exosquelette inférieur peuvent s'appuyer.

Selon certains modes de réalisation, le dispositif orthopédique peut comprendre en outre un sac à dos dans lequel l'actionneur émetteur est installé.

Selon certains modes de réalisation, chaque exosquelette dudit dispositif orthopédique est formé d'au moins un matériau choisi dans le groupe formé des matériaux polymères, des matériaux composites, et de leurs mélanges.

Selon certains modes de réalisation, chaque exosquelette dudit dispositif orthopédique est formé d'au moins un matériau composite comprenant au moins un renfort sous forme de fibres et au moins un matériau polymère au sein duquel s'étendent les fibres.

Un aspect non revendiqué de l'invention concerne également un procédé de mise en œuvre d'un tel dispositif orthopédique, en particulier, un procédé de mise en œuvre d'un tel dispositif orthopédique dans lequel :
- on assemble ledit exosquelette supérieur et ledit exosquelette inférieur audit actionneur récepteur,
- on met en place ledit exosquelette supérieur et ledit exosquelette inférieur sur un membre inférieur humain de façon à équiper un utilisateur de l'actionneur récepteur dudit dispositif orthopédique,
- on installe ledit actionneur émetteur sur ledit utilisateur, ledit actionneur émetteur étant relié audit actionneur récepteur,
- on démarre l'unité électronique et on utilise l'interface homme machine afin de choisir les paramètres de fonctionnement voulus.

Selon l'invention, l'actionneur émetteur peut être porté par l'utilisateur du dispositif orthopédique selon l'invention de différentes façons permettant de déporter ledit actionneur émetteur dudit actionneur récepteur et de l'articulation concernée. Selon certains modes de réalisation, l'actionneur émetteur peut être installé (ainsi que l'unité électronique) dans une sacoche pouvant être portée autour de la taille, près du bassin de l'utilisateur dudit dispositif orthopédique, ou encore dans un sac à dos porté par l'utilisateur dudit dispositif orthopédique, ou encore fixé à la ceinture de l'utilisateur dudit dispositif orthopédique.

Un aspect non revendiqué de l'invention concerne également un procédé de fabrication d'un tel dispositif orthopédique.

L'invention concerne également un dispositif orthopédique caractérisé, en combinaison ou non, par tout ou partie des caractéristiques mentionnées ci-dessus ou ci-après. Quelle que soit la présentation formelle qui en est donnée, sauf indication contraire explicite, les différentes caractéristiques mentionnées ci-dessus ou ci-après ne doivent pas être considérées comme étroitement ou inextricablement liées entre elles, l'invention pouvant concerner l'une seulement de ces caractéristiques structurelles ou fonctionnelles, ou une partie seulement de ces caractéristiques structurelles ou fonctionnelles, ou une partie seulement de l'une de ces caractéristiques structurelles ou fonctionnelles, ou encore tout groupement, combinaison ou juxtaposition de tout ou partie de ces caractéristiques structurelles ou fonctionnelles.

### Brève description des figures

D'autres buts, caractéristiques et avantages de l'invention apparaîtront à la lecture de la description détaillée suivante de certains modes de réalisation possibles, donnée à titre non limitatif et qui se réfère aux figures annexées dans lesquelles :
[Fig. 1] représente une vue schématique d'un dispositif orthopédique selon l'invention ;
[Fig. 2] représente une vue schématique d'un dispositif orthopédique selon l'invention (sans exosquelette).
[Fig. 3] représente une vue schématique d'un dispositif orthopédique selon l'invention (sans exosquelette),
[Fig. 4] Les figures 4a et 4b représentent des vues schématiques d'un actionneur récepteur d'un dispositif orthopédique selon l'invention,
[Fig. 5] Les figures 5a et 5b représentent des vues schématiques d'un actionneur récepteur d'un dispositif orthopédique selon l'invention,
[Fig. 6] Les figures 6a et 6b représentent des vues schématiques d'un actionneur émetteur d'un dispositif orthopédique selon un mode de réalisation non revendiqué,
[Fig. 7] Les figures 7a et 7b représentent des vues schématiques d'un actionneur émetteur d'un dispositif orthopédique selon un mode de réalisation non revendiqué,
[Fig. 8] représente une vue schématique de l'architecture de contrôle d'un dispositif orthopédique selon l'invention,
[Fig. 9] représente une vue schématique de profil d'un utilisateur équipé d'un dispositif orthopédique selon l'invention.

### Description de certains modes de réalisation

Dans les figures illustratives de l'invention, données uniquement à titre non limitatif, les proportions ne sont pas nécessairement respectées, et ceci uniquement aux fins de clarté de l'exposé.

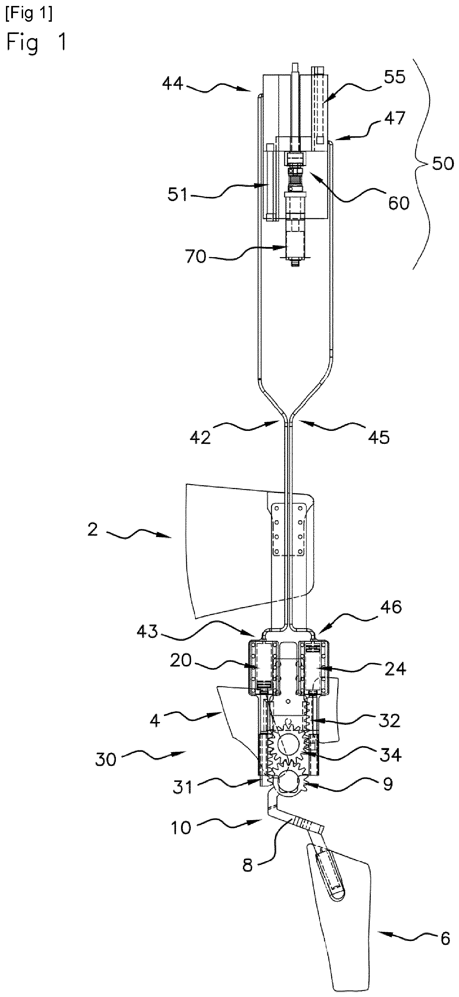

Le dispositif orthopédique illustré aux figures 1, 2 et 3 comprend un exosquelette supérieur 2, 4 et un exosquelette inférieur 6 articulés l'un par rapport à l'autre par un organe 10 de liaison pivot. Ici, comme on peut le voir sur la figure 1, l'exosquelette supérieur comprend une portion 2 disposée sur le haut de la cuisse et une portion 4 entourant la partie basse de la cuisse, au-dessus du genou. L'exosquelette inférieur 6 comprend une portion en appui sur le mollet du membre inférieur équipé par le dispositif orthopédique.

L'organe de liaison pivot comprend une roue 9 dentée fixée à un levier 8 relié à l'exosquelette inférieur 6. Le levier 8 peut se présenter sous la forme d'une tige métallique coudée ou d'une barre présentant plusieurs portions courbes et/ou rectilignes juxtaposées entre elles.

Le dispositif orthopédique comprend un dispositif de transmission récepteur 30 adapté pour pouvoir transmettre un mouvement et un effort à l'organe 10 de liaison pivot. Le dispositif de transmission récepteur 30 tel qu'illustré en figure 1 est du type crémaillère/pignon.

Le dispositif orthopédique comprend un actionneur récepteur accouplé au dispositif de transmission récepteur 30 pour pouvoir entraîner en rotation l'organe 10 de liaison pivot. L'actionneur récepteur comprend un premier vérin 20 récepteur hydraulique et un deuxième vérin 24 récepteur hydraulique.

Le premier vérin 20 récepteur hydraulique entraîne en translation une première crémaillère 31 engrenée avec une première roue dentée 34. Le deuxième vérin 24 récepteur hydraulique entraîne en translation une deuxième crémaillère 32 également engrenée avec la roue dentée 34. La roue dentée 9 de l'organe de liaison pivot 10 est également engrenée avec la roue dentée 34. La première crémaillère 31 et la deuxième crémaillère 32 s'étendent longitudinalement parallèlement l'une à l'autre. Le dispositif de transmission récepteur 30 présente donc dans ce cas une configuration à double pignon et à double crémaillère.

Le premier vérin 20 récepteur hydraulique et le deuxième vérin 24 récepteur hydraulique sont respectivement actionnés via deux conduits 42 et 45 de guidage de fluide sous pression. Les deux conduits 42 et 45 hydraulique de guidage de fluide sous pression sont alimentés par un actionneur émetteur 50, ce dernier étant déporté physiquement par rapport à l'actionneur récepteur 30 ce qui permet donc de le déporter par rapport à la jambe et au genou de l'utilisateur. Chaque conduit 42, 45 hydraulique de guidage de fluide est relié respectivement au premier vérin 20 récepteur hydraulique et deuxième vérin 24 récepteur hydraulique par un orifice de communication de fluide 43 et 46 de chaque vérin 20, 24 récepteur hydraulique, comme on peut le voir sur les figures 1 à 3.

L'actionneur émetteur 50 comprend un premier vérin émetteur hydraulique 51 et un deuxième vérin émetteur hydraulique 55 reliés à un dispositif de transmission émetteur 60 adapté pour pouvoir transmettre un mouvement et un effort aux vérins émetteur 51, 55. Le dispositif de transmission émetteur 60, représenté en figures 1, 2 et 3, est du type vis/écrou.

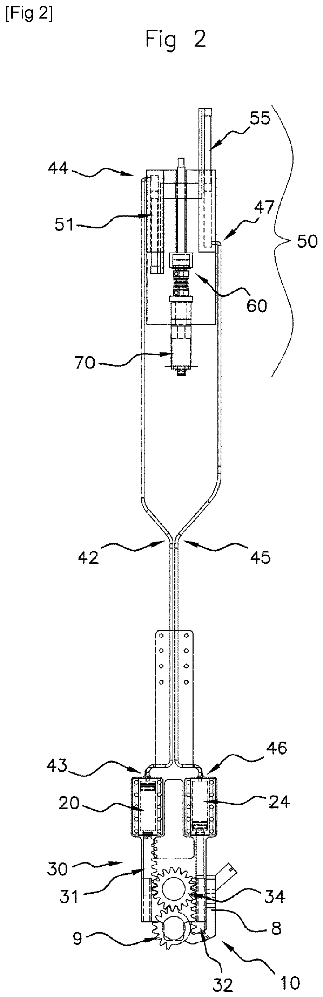

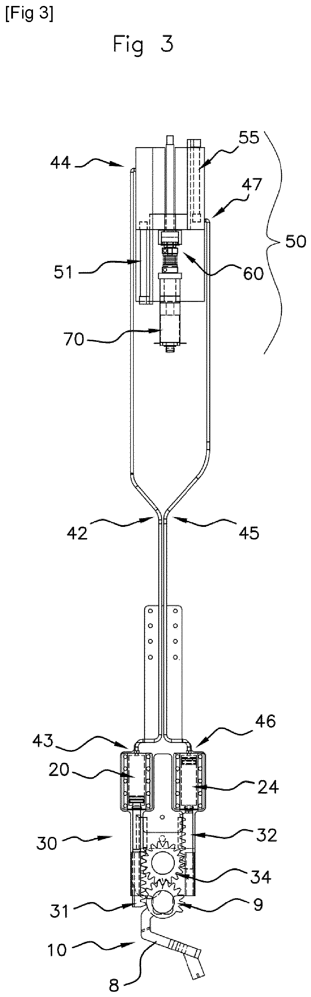

L'actionneur émetteur 50 comprend un dispositif moteur 70 accouplé au dispositif de transmission émetteur 60 afin de pouvoir entraîner les vérins émetteur hydrauliques 51, 55 hydrauliques. Le dispositif de transmission émetteur 60, représenté en figures 1, 2 et 3, est du type vis/écrou. Un rotor du dispositif moteur 70 est couplé à la vis du dispositif de transmission émetteur 60, cette vis coopérant avec le taraudage d'un écrou relié mécaniquement aux pistons respectifs du premier vérin émetteur hydraulique 51 et du deuxième vérin émetteur hydraulique 55. Le premier vérin émetteur hydraulique 51 et le deuxième vérin émetteur hydraulique 55 sont installés en opposition de sorte que lorsque l'écrou du dispositif de transmission émetteur 60 se déplace en translation par l'action de rotation de la vis du dispositif de transmission émetteur 60, le piston du premier vérin émetteur hydraulique 51 rentre dans son cylindre et le piston du deuxième vérin émetteur hydraulique 55 sort de son cylindre et vice versa. Ainsi, dépendamment du sens de translation de l'écrou du dispositif de transmission émetteur 60, un delta de pression est induit dans le premier vérin émetteur hydraulique 51 ou le deuxième vérin émetteur hydraulique 55. Le premier vérin émetteur hydraulique 51 et le deuxième vérin émetteur hydraulique 55 alimentent à leur tour en fluide sous pression les conduits hydrauliques 42 et 45 de guidage de fluide. Chaque conduit 42, 45 de guidage de fluide est relié respectivement au premier vérin émetteur hydraulique 51 et au deuxième vérin émetteur hydraulique 55 par un orifice de communication de fluide 44, 47 de chaque vérin émetteur 51, 55, comme on peut le voir sur les figures 1 à 3. Les figures 2 et 3 illustrent respectivement une flexion et une extension de la jambe à laquelle serait fixée le dispositif orthopédique.

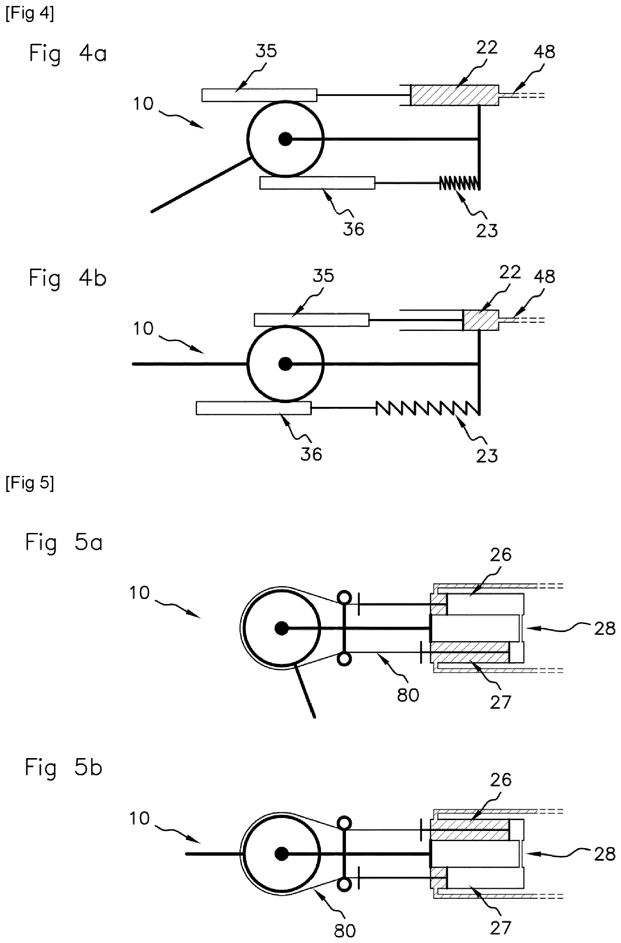

Dans un autre mode de réalisation d'un dispositif orthopédique selon l'invention, l'actionneur récepteur, tel qu'illustré en figures 4a et 4b peut comprendre un premier vérin 22 récepteur hydraulique et un ressort 23. Dans ce cas, le dispositif orthopédique ne comprend qu'un unique conduit 48 de guidage de fluide. Le dispositif de transmission d'un tel actionneur récepteur illustré aux figures 4a et 4b est du type pignon/crémaillère. Le premier vérin 22 récepteur hydraulique entraîne en déplacement une crémaillère 35 et le ressort 23 entraîne en déplacement une crémaillère 36. Les figures 4a et 4b illustrent respectivement une flexion et une extension de la jambe à laquelle serait fixée le dispositif orthopédique. La barre en gras en forme de T renversé sur les figures 4a, 4b reliant le vérin 22 récepteur hydraulique, le ressort 23 et l'organe de liaison 10 schématise une pièce fixe de type stator reliant ces éléments du dispositif orthopédique (il s'agit en pratique par exemple d'une platine à laquelle est également fixé l'exosquelette supérieur).

Dans un autre mode de réalisation d'un dispositif orthopédique selon l'invention, l'actionneur récepteur, tel qu'illustré en figures 5a et 5b peut comprendre un premier vérin 26 récepteur hydraulique à double effet et un deuxième vérin 27 récepteur hydraulique à double effet. Le premier vérin 26 récepteur hydraulique et le deuxième vérin 27 récepteur hydraulique sont reliés entre eux par un tube 28 de communication de fluide. Le dispositif de transmission d'un tel actionneur récepteur illustré aux figures 5a et 5b est du type comprenant un câble 80 relié par une extrémité au piston du premier vérin 26 récepteur hydraulique et par une autre extrémité au deuxième vérin 27 récepteur hydraulique. Les figures 5a et 5b illustrent respectivement une flexion et une extension de la jambe à laquelle serait fixée le dispositif orthopédique.

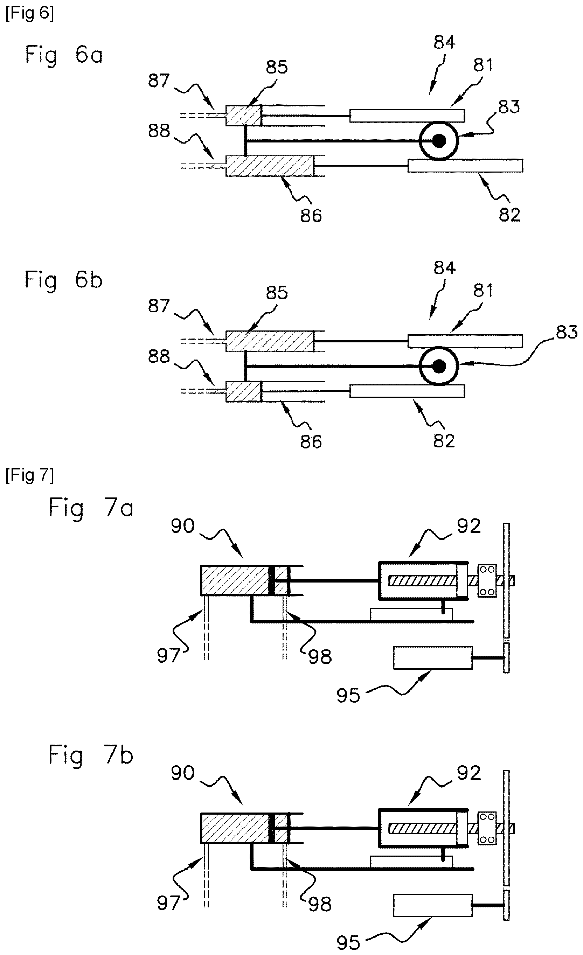

Dans un mode de réalisation d'un dispositif orthopédique non revendiqué, l'actionneur émetteur, tel qu'illustré en figures 6a et 6b (configurations correspondantes à une flexion et à une extension), comprend un premier vérin émetteur hydraulique 85, un deuxième vérin émetteur hydraulique 86 et un dispositif 84 de transmission mécanique de type pignon/crémaillère comprenant deux crémaillères 81, 82 actionnées par une roue dentée 83 comprenant un dispositif moteur l'entraînant en rotation. Chaque vérin hydraulique 85, 86 est respectivement relié à un conduit hydraulique 87, 88 de guidage de fluide.

Dans un autre mode de réalisation d'un dispositif orthopédique non revendiqué, l'actionneur émetteur tel qu'illustré en figures 7a et 7b (configurations correspondantes à une flexion et à une extension) comprend un vérin à double effet 90 reliés à des conduits hydrauliques 97, 98 de guidage de fluide, et un dispositif de transmission émetteur de type vis/écrou 92 couplé à un dispositif moteur 95.

Selon l'invention, le dispositif orthopédique peut comprendre des capteurs 106 tels que des accéléromètres, des capteurs de position, des capteurs de pression, des capteurs de couple de torsion, des capteurs de force ou encore des capteurs de déformation.

Le dispositif orthopédique comprend une unité électronique 100 et une interface homme/machine 105. L'interface homme/machine 105 peut comprendre tout type d'écran d'affichage (ordinateur, téléphone portable, tablette, etc...) et permet à un utilisateur de définir les paramètres de fonctionnement du dispositif orthopédique. De tels capteurs et les données communiquées à l'unité électronique 100 permettent de détecter une intention de mouvement de la part de l'utilisateur équipé du dispositif orthopédique.

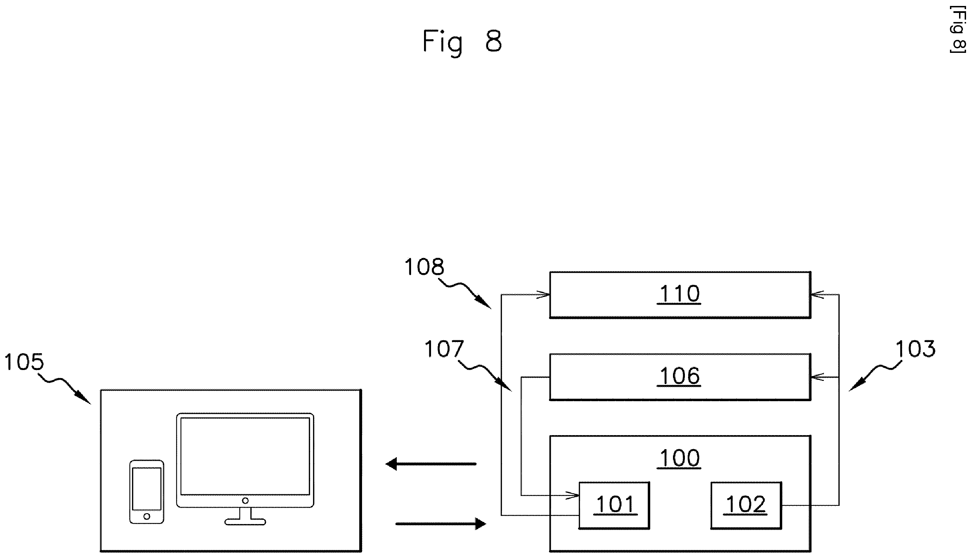

La figure 8 illustre l'architecture de contrôle d'un dispositif orthopédique selon l'invention. L'unité électronique 100 comprend une unité 101 de contrôle de l'exosquelette (c'est-à-dire l'exosquelette supérieur et l'exosquelette inférieur) et une unité 102 d'alimentation en énergie électrique. L'unité 102 d'alimentation fournit l'énergie électrique nécessaire aux capteurs 106 et à l'actionneur émetteur schématisé par la case 110 en figure 8, comme illustré par la flèche 103. Comme illustré par la double flèche en figure 8 entre l'interface homme/machine 105 et l'unité électronique 100, le choix d'un mode opératoire par exemple est communiqué à l'unité 101 de contrôle de l'exosquelette par l'interface homme/machine 105 et des données représentatives du fonctionnement du dispositif orthopédique (en particulier les mesures réalisées par les capteurs) sont transmises à l'interface homme/machine 105. La flèche 108 symbolise les commandes données par l'unité 101 de contrôle de l'exosquelette à l'actionneur émetteur schématisé par la case 110. Et la flèche 107 symbolise les données envoyées en retour par les capteurs 106 (ou autres instruments) à l'unité 101 de contrôle de l'exosquelette.

Le dispositif orthopédique est commandé à l'aide d'une boucle de rétroaction entre le dispositif moteur de l'actionneur émetteur et les signaux transmis par lesdits capteurs. Lorsque le dispositif orthopédique détecte une intention de mouvement de la jambe de la part de l'utilisateur, une loi de contrôle haut niveau de l'unité électronique définit une commande (un couple ou une rotation prédéterminée) à appliquer à l'organe de liaison pivot. Une loi de contrôle bas niveau de l'unité électronique asservit ensuite le mécanisme électrohydraulique pour appliquer la commande prédéfinie à l'organe de liaison pivot du dispositif orthopédique.

Dans le mode de réalisation d'un dispositif orthopédique représentée aux figures 1 à 3, pour un mouvement d'extension de la jambe (figure 3), le dispositif moteur 70 tourne dans le sens contraire des aiguilles d'une montre, ce qui déplace l'écrou du dispositif de transmission émetteur 60 de l'actionneur émetteur vers le dispositif moteur 70. Cela conduit à un effet double sur les vérins émetteurs 51 et 55 de l'actionneur émetteur : un delta de pression relatif négatif est induit dans l'un tandis qu'un delta de pression relatif positif est induit dans l'autre. Les conduits 42 et 45 hydrauliques de guidage de fluide transmettent la variation de pression aux vérins 20, 24 récepteurs, ce qui permet la transmission d'une force aux crémaillères 31, 32 et ainsi la transmission d'un couple aux roues dentées 9 et 34. L'exosquelette inférieur 6 est entraîné en rotation autour de l'axe de pivot de l'organe de liaison pivot dans le sens des aiguilles d'une montre et contribue ainsi à l'extension de la jambe de l'utilisateur.

Dans le mode de réalisation d'un dispositif orthopédique représentée aux figures 1 à 3, pour un mouvement de flexion de la jambe (figure 2), le dispositif moteur 70 tourne dans le sens des aiguilles d'une montre, ce qui déplace l'écrou du dispositif de transmission émetteur 60 de l'actionneur émetteur par rapport au dispositif moteur 70. Cela conduit à un effet double sur les vérins émetteurs 51 et 55 de l'actionneur émetteur : un delta de pression relatif positif est induit dans l'un tandis qu'un delta de pression relatif négatif est induit dans l'autre. Les conduits 42 et 45 hydrauliques de guidage de fluide transmettent la variation de pression aux vérins 20, 24 récepteurs, ce qui permet la transmission d'une force aux crémaillères 31, 32 et ainsi la transmission d'un couple aux roues dentées 9, 34. L'exosquelette inférieur 6 est entraîné en rotation autour de l'axe de pivot de l'organe 10 de liaison pivot dans le sens inverse des aiguilles d'une montre et contribue ainsi à la flexion de la jambe de l'utilisateur.

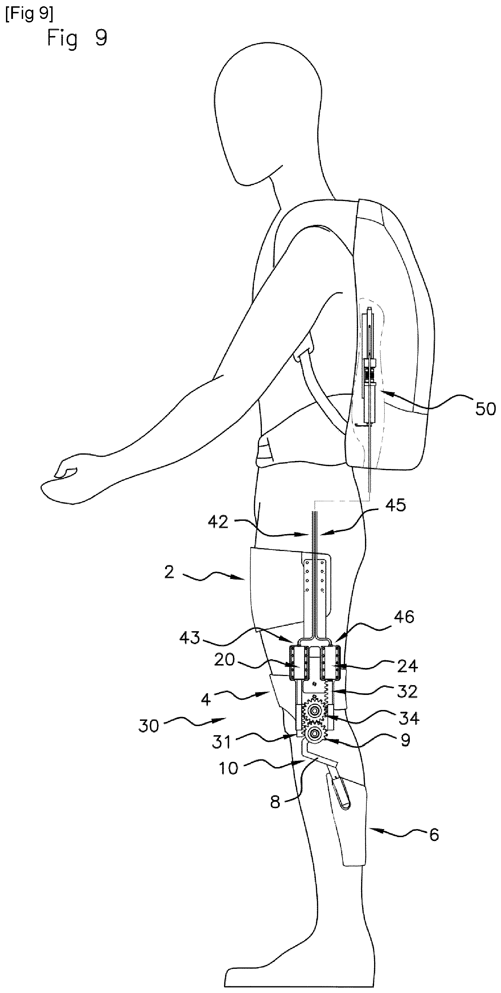

La figure 9 illustre un dispositif orthopédique selon l'invention équipant un utilisateur. Dans le mode de réalisation illustré, le dispositif orthopédique correspondant au mode de réalisation illustré aux figures 1 à 3 équipe un utilisateur portant un sac à dos dans lequel est disposé l'actionneur émetteur. Il est à noter qu'il peut être prévu que chaque actionneur soit disposé dans un carter (non représenté) afin de faciliter leur manipulation et de les protéger.

Les portions de l'exosquelette supérieur 2, 4 et de l'exosquelette inférieur 6 telles que représentées en figure 1 peuvent faire l'objet de nombreuses autres formes ou être disposées en appui contre d'autres parties (en alternative ou en combinaison) du membre inférieur. Elles peuvent comprendre des bandes de réglages ou tout autre dispositif de réglage et/ou de fixation de façon à pouvoir s'adapter à tout type de morphologie. Il est également possible de réaliser sur mesure pour chaque utilisateur chaque portion de l'exosquelette supérieur 2, 4 et de l'exosquelette inférieur 6 en fonction des pathologies ou de la morphologie de chaque utilisateur.

L'invention peut faire l'objet de nombreuses variantes et applications autres que celles décrites ci-dessus. En particulier, il va de soi que sauf indication contraire les différentes caractéristiques structurelles et fonctionnelles de chacun des modes de réalisation décrits ci-dessus ne doivent pas être considérées comme combinées et/ou étroitement et/ou inextricablement liées les unes aux autres, mais au contraire comme de simples juxtapositions. En outre, les caractéristiques structurelles et/ou fonctionnelles des différents modes de réalisation décrits ci-dessus peuvent faire l'objet en tout ou partie de toute juxtaposition différente ou de toute combinaison différente.

En particulier, cette description est donnée à titre d'exemple illustratif uniquement. L'homme du métier pourra y apporter de nombreuses modifications, outre les variantes évoquées au cours de la description ci-dessus, sans sortir de la portée de l'invention. Un dispositif orthopédique selon l'invention peut par exemple également se présenter sous la forme d'une orthèse de hanche ou d'une orthèse cruro-pédieuse.

## Revendications

1. Dispositif orthopédique comprenant :
- au moins une portion, dite exosquelette supérieur (2, 4), adaptée pour former un appui en contact avec une première partie du corps humain,
- au moins une portion, dite exosquelette inférieur (6), adaptée pour former un appui en contact avec une deuxième partie du corps humain,
- un actionneur, dit actionneur récepteur, comprenant :
∘ un organe (10) de liaison pivot présentant au moins un axe de pivot, ledit exosquelette supérieur et ledit exosquelette inférieur étant articulés l'un par rapport à l'autre par ledit organe de liaison pivot,
∘ un dispositif de transmission récepteur (30) adapté pour pouvoir transmettre un mouvement à l'organe de liaison pivot, et
∘ au moins un premier vérin (20) hydraulique, dit premier vérin récepteur, accouplé au dispositif de transmission récepteur pour pouvoir entraîner en rotation l'organe de liaison pivot,
- un actionneur, dit actionneur émetteur (50), comprenant :
∘ un dispositif moteur (70),
∘ un dispositif de transmission émetteur (60) de type vis/écrou accouplé au dispositif moteur, et
∘ deux vérins hydrauliques, dit respectivement premier vérin émetteur (51) et deuxième vérin émetteur (55), comprenant chacun un piston et un cylindre associé, et
∘ deux conduits hydrauliques de guidage de fluide sous pression (42, 45) respectivement reliés au premier vérin émetteur (51) et au deuxième vérin émetteur (55), et adaptés pour permettre une transmission hydraulique de mouvement de l'actionneur émetteur (50) à l'actionneur récepteur,
**caractérisé par le fait que** :
un rotor du dispositif moteur (70) étant couplé à la vis du dispositif de transmission émetteur (60), cette vis coopérant avec le taraudage d'un écrou relié mécaniquement aux pistons respectifs du premier vérin émetteur hydraulique (51) et du deuxième vérin émetteur hydraulique (55); et le premier vérin émetteur (51) et le deuxième vérin émetteur (55) sont installés en opposition en étant reliés mécaniquement à l'écrou du dispositif de transmission émetteur (60) de telle sorte que lorsque ledit écrou se déplace en translation par l'action de rotation de la vis du dispositif de transmission émetteur (60) le piston du premier vérin émetteur (51) rentre dans le cylindre associé et le piston du deuxième vérin émetteur hydraulique (55) sort du cylindre associé, et vice versa.

2. Dispositif selon la revendication 1, dans lequel les premier et deuxième vérins émetteurs (51, 55) de l'actionneur émetteur sont choisis parmi les vérins à simple effet et les vérins à double effet.

3. Dispositif selon l'une quelconque des revendications 1 et 2, dans lequel le dispositif moteur (70) de l'actionneur émetteur est un moteur électrique, un moteur hydraulique ou un moteur pneumatique.

4. Dispositif selon l'une quelconque des revendications 1 à 3, dans lequel l'actionneur récepteur comprend un deuxième vérin, dit deuxième vérin récepteur (24), le premier vérin récepteur et ledit deuxième vérin récepteur étant respectivement reliés à chacun des deux conduits (42, 45) hydrauliques de guidage de fluide sous pression (42, 45).

5. Dispositif selon la revendication 4 dans lequel les premier et deuxième vérins récepteurs (20, 24) de l'actionneur récepteur sont des vérins à simple effet ou à double effet.

6. Dispositif selon l'une quelconque des revendications 1 à 5, dans lequel le dispositif de transmission récepteur (30) est choisi dans le groupe formé des transmissions mécaniques adaptées pour pouvoir transformer un mouvement de translation transmis par l'actionneur récepteur en un mouvement de rotation de l'organe de liaison pivot.

7. Dispositif selon l'une quelconque des revendications 1 à 6 dans lequel le dispositif de transmission récepteur (30) est choisi dans le groupe formé des transmissions mécaniques à câbles et/ou courroies, des transmissions de type bielle/manivelle et des transmissions mécaniques de type pignon/crémaillère.

8. Dispositif selon l'une quelconque des revendications 1 à 7 comprenant en outre une unité électronique (100) pour définir une commande à appliquer à l'organe de liaison pivot et une interface homme/machine (105) pour permettre à un utilisateur de définir des paramètres de fonctionnement du dispositif orthopédique.

9. Dispositif selon l'une quelconque des revendications 1 à 8 comprenant en outre au moins un capteur (106) choisi dans le groupe formé des accéléromètres, des capteurs de position, des capteurs de pression, des capteurs de couple de torsion, des capteurs de force et des capteurs de déformation.

10. Dispositif selon l'une quelconque des revendications 1 à 9, dans lequel l'organe de liaison pivot comprend une roue dentée et un levier qui est relié à l'exosquelette inférieur et qui se présente sous la forme d'une barre ayant plusieurs portions courbes et/ou rectilignes juxtaposées entre elles, ledit organe de liaison pivot étant adapté pour autoriser une rotation d'au moins deux portions de ladite barre métallique l'une par rapport à l'autre autour d'un axe orthogonal à l'axe de pivot de l'organe (10) de liaison pivot de sorte que ledit organe de liaison pivot peut présenter un ou plusieurs degré(s) de réglage hors plan pour s'adapter à la démarche d'un utilisateur.

11. Dispositif selon l'une quelconque des revendications 1 à 10, dans lequel l'organe (10) de liaison pivot est un organe de liaison pivot polycentrique.

12. Dispositif selon la revendication 11 dans lequel l'organe de liaison pivot polycentrique est adapté à la cinématique d'une articulation entre la première partie et la deuxième partie du corps humain au contact desquelles l'exosquelette supérieur (2, 4) et l'exosquelette inférieur (6) peuvent s'appuyer.

13. Dispositif selon l'une quelconque des revendications 1 à 12, comprenant en outre un sac à dos dans lequel l'actionneur émetteur est installé.

## Patentansprüche

1. Orthopädische Vorrichtung, umfassend:
- mindestens einen Abschnitt, das sogenannte obere Exoskelett (2, 4), der angepasst ist, um eine Anlage in Kontakt mit einem ersten Teil des menschlichen Körpers zu bilden,
- mindestens einen Abschnitt, das sogenannte untere Exoskelett (6), der angepasst ist, um eine Anlage in Kontakt mit einem zweiten Teil des menschlichen Körpers zu bilden,
- einen Aktuator, der sogenannte Nehmeraktuator, umfassend:
∘ ein Schwenkverbindungsorgan (10), das mindestens eine Schwenkachse aufweist, wobei das obere Exoskelett und das untere Exoskelett durch das Schwenkverbindungsorgan gelenkig miteinander verbunden sind,
∘ eine Nehmerübertragungsvorrichtung (30), die angepasst ist, um eine Bewegung auf das Schwenkverbindungsorgan zu übertragen, und
∘ mindestens einen ersten Hydraulikzylinder (20), der sogenannte erste Nehmerzylinder, der mit der Nehmerübertragungsvorrichtung gekoppelt ist, um das Schwenkverbindungsorgan in Drehung versetzen zu können,
- einen Aktuator, der sogenannte Geberaktuator (50), umfassend:
∘ eine Motorvorrichtung (70),
∘ eine Geberübertragungsvorrichtung (60) vom Typ Schraube/Mutter, die mit der Motorvorrichtung gekoppelt ist, und
∘ zwei Hydraulikzylinder, der sogenannte erste Geberzylinder (51) und zweite Geberzylinder (55), jeweils umfassend einen Kolben und einen assoziierten Zylinder, und
∘ zwei hydraulische Druckfluidführungsleitungen (42, 45), die jeweils mit dem ersten Geberzylinder (51) und dem zweiten Geberzylinder (55) verbunden und angepasst sind, um eine hydraulische Bewegungsübertragung von dem Geberaktuator (50) auf den Empfangsaktuator zu ermöglichen, **dadurch gekennzeichnet, dass**:
ein Rotor der Motorvorrichtung (70) mit der Schraube der Geberübertragungsvorrichtung (60) gekoppelt ist, wobei diese Schraube mit dem Innengewinde einer Mutter zusammenwirkt, die mechanisch mit den jeweiligen Kolben des ersten hydraulischen Geberzylinders (51) und des zweiten hydraulischen Geberzylinders (55) verbunden ist; und
der erste Geberzylinder (51) und der zweite Geberzylinder (55) gegenüberliegend installiert sind, indem sie mechanisch mit der Mutter der Geberübertragungsvorrichtung (60) verbunden sind, sodass, wenn sich die Mutter durch die Drehwirkung der Schraube der Geberübertragungsvorrichtung (60) translatorisch bewegt, der Kolben des ersten Geberzylinders (51) in den assoziierten Zylinder einfährt und der Kolben des zweiten hydraulischen Geberzylinders (55) aus dem assoziierten Zylinder ausfährt, und umgekehrt.

2. Vorrichtung nach Anspruch 1, wobei der erste und der zweite Geberzylinder (51, 55) des Geberaktuators ausgewählt sind aus einfachwirkenden Zylindern und doppeltwirkenden Zylindern.

3. Vorrichtung nach einem der Ansprüche 1 und 2, wobei die Motorvorrichtung (70) des Geberaktuators ein Elektromotor, ein Hydraulikmotor oder ein Luftmotor ist.

4. Vorrichtung nach einem der Ansprüche 1 bis 3, wobei der Nehmeraktuator einen zweiten Zylinder umfasst, der sogenannte zweite Nehmerzylinder (24), wobei der erste Nehmerzylinder und der zweite Nehmerzylinder jeweils mit jeder von zwei hydraulischen Leitungen (42, 45) zur Druckfluidführung (42, 45) verbunden sind.

5. Vorrichtung nach Anspruch 4, wobei der erste und der zweite Nehmerzylinder (20, 24) des Nehmeraktuators einfachwirkende oder doppeltwirkende Zylinder sind.

6. Vorrichtung nach einem der Ansprüche 1 bis 5, wobei die Nehmerübertragungsvorrichtung (30) ausgewählt ist aus der Gruppe, bestehend aus mechanischen Übertragungen, die angepasst sind, um eine Translationsbewegung, die von dem Nehmeraktuator übertragen wird, in eine Drehbewegung des Schwenkverbindungsorgans umzuwandeln.

7. Vorrichtung nach einem der Ansprüche 1 bis 6, wobei die Nehmerübertragungsvorrichtung (30) ausgewählt ist aus der Gruppe, bestehend aus mechanischen Seil- und/oder Riemenübertragungen, Übertragungen vom Typ Pleuelstange/Kurbel und mechanischen Übertragungen vom Typ Ritzel/Zahnstange.

8. Vorrichtung nach einem der Ansprüche 1 bis 7, ferner umfassend eine Elektronikeinheit (100) zum Definieren eines Befehls, der auf das Schwenkverbindungsorgan angewendet werden soll, und eine Mensch-Maschine-Schnittstelle (105), die es einem Benutzer ermöglicht, Betriebsparameter der orthopädischen Vorrichtung zu definieren.

9. Vorrichtung nach einem der Ansprüche 1 bis 8, ferner umfassend mindestens einen Sensor (106), der ausgewählt ist aus der Gruppe, bestehend aus Beschleunigungsmessern, Positionssensoren, Drucksensoren, Drehmomentsensoren, Kraftsensoren und Verformungssensoren.

10. Vorrichtung nach einem der Ansprüche 1 bis 9, wobei das Schwenkverbindungsorgan ein Zahnrad und einen Hebel umfasst, der mit dem unteren Exoskelett verbunden ist und in Form einer Stange vorliegt, die mehrere nebeneinander liegende gekrümmte und/oder gerade Abschnitte aufweist, wobei das Schwenkverbindungsorgan angepasst ist, um eine Drehung von mindestens zwei Abschnitten der Metallstange in Bezug aufeinander um eine Achse orthogonal zu der Schwenkachse des Schwenkverbindungsorgans (10) zuzulassen, sodass das Schwenkverbindungsorgan einen oder mehrere Grad an ebenenversetzte Einstellung aufweisen kann, um sich an den Gang eines Benutzers anzupassen.

11. Vorrichtung nach einem der Ansprüche 1 bis 10, wobei das Schwenkverbindungsorgan (10) ein polyzentrisches Schwenkverbindungsorgan ist.

12. Vorrichtung nach Anspruch 11, wobei das polyzentrische Schwenkverbindungsorgan an die Kinematik eines Gelenks zwischen dem ersten Teil und dem zweiten Teil des menschlichen Körpers angepasst ist, an deren Kontakt das obere Exoskelett (2, 4) und das untere Exoskelett (6) anliegen können.

13. Vorrichtung nach einem der Ansprüche 1 bis 12, ferner umfassend einen Rucksack umfasst, in dem der Geberaktuator installiert ist.

## Claims

1. An orthopaedic device comprising:
- at least one portion, referred to as upper exoskeleton (2, 4), suitable for forming an abutment in contact with a first part of the human body,
- at least one portion, referred to as lower exoskeleton (6), suitable for forming an abutment in contact with a second part of the human body,
- an actuator, referred to as receiving actuator, comprising:
∘ a pivot-connection member (10) having at least one pivot axis, said upper exoskeleton and said lower exoskeleton being hinged with respect to one another via said pivot-connection member,
∘ a receiving transmission device (30) designed to be able to transmit a movement to the pivot-connection member, and
∘ at least a first hydraulic cylinder (20), referred to as first receiving cylinder, coupled to the receiving transmission device so as to be able to rotate the pivot-connection member,
- an actuator, referred to as emitting actuator (50), comprising:
∘ a motor device (70),
∘ an emitting transmission device (60) of the screw/nut type coupled to the motor device, and
∘ two hydraulic cylinders, referred to respectively as first emitting cylinder (51) and second emitting cylinder (55), each comprising a piston and an associated cylinder, and
- two pressurized-fluid-guiding hydraulic lines (42, 45) connected respectively to the first emitting cylinder (51) and to the second emitting cylinder (55), and adapted to allow a hydraulic transmission of movement from the emitting actuator (50) to the receiving actuator,
**characterized in that**:
a rotor of the motor device (70) being coupled to the screw of the emitting transmission device (60), this screw cooperating with the tapping of a nut mechanically connected to respective pistons of the first hydraulic emitting cylinder (51) and of the second hydraulic emitting cylinder (55) ; and the first emitting cylinder (51) and the second emitting cylinder (55) are installed in opposition by being mechanically connected to the nut of the emitting transmission device (60) such that when said nut is moved in translation by the rotational action of the screw of the emitting transmission device (60), the piston of the first emitting cylinder (51) enters the associated cylinder and the piston of the second hydraulic emitting cylinder (55) leaves the associated cylinder, and vice versa.

2. The device according to claim 1, wherein the first and second emitting cylinders (51, 55) of the emitting actuator are selected from among single-acting cylinders and double-acting cylinders.

3. The device according to any one of claims 1 and 2, wherein the motor device (70) of the emitting actuator is an electric motor, a hydraulic motor or a pneumatic motor.

4. The device according to any one of claims 1 to 3, wherein the receiving actuator comprises a second cylinder, referred to as second receiving cylinder (24), the first receiving cylinder and said second receiving cylinder being connected respectively to each of the two pressurized-fluid-guiding hydraulic lines (42 ,45).

5. The device according to claim 4, wherein the first and second receiving cylinders (20, 24) of the receiving actuator are single-acting cylinders or double-acting cylinders.

6. The device according to any one of claims 1 to 5, wherein the receiving transmission device (30) is selected from the group formed by mechanical transmissions adapted to be able to transform a translational movement transmitted by the receiving actuator into a rotational movement of the pivot-connection member.

7. The device according to any one of claims 1 to 6, wherein the receiving transmission device (30) is selected from the group formed by cable and/or belt mechanical transmissions, transmissions of the rod-crank type, and mechanical transmissions of the pinion-rack type.

8. The device according to any one of claims 1 to 7, further comprising an electronic unit (100) for defining a command to be applied to the pivot-connection member and a human/machine interface (105) for allowing a user to define operating parameters of the orthopaedic device.

9. The device according to any one of claims 1 to 8, further comprising at least one sensor (106) selected from the group formed by accelerometers, position sensors, pressure sensors, twisting-torque sensors, force sensors and deformation sensors.

10. The device according to any one of claims 1 to 10, wherein the pivot-connection member comprises a toothed wheel and a lever which is connected to the lower exoskeleton and is in the form of a bar having multiple curved and/or straight portions juxtaposed with one another, said pivot-connection member being adapted to allow at least two portions of said metal bar to rotate with respect to one another about an axis orthogonal to the pivot axis of the pivot-connection member (10) such that said pivot-connection member may exhibit one or more degrees of adjustment out of the plane, in order to be able to better adapt to the user's gait.

11. The device according to any one of claims 1 to 10, wherein the pivot-connection member (10) is a polycentric pivot-connection member.

12. The device according to claim 11, wherein the polycentric pivot-connection member is adapted to the kinematics of a joint between the first part and the second part of the human body in contact with which the upper exoskeleton (2, 4) and the lower exoskeleton (6) can rest.

13. The device according to any one of claims 1 to 12, further comprising a backpack in which the emitting actuator is installed.
